# EUROPEAN PATENT SPECIFICATION

(11) **EP 3 174 570 B1**
(45) Date of publication and mention of the grant of the patent: **17.03.2021**
(21) Application number: 15753798.6
(22) Date of filing: 30.07.2015
(51) Int. Cl.: A61M 1/00, G16H 20/40, G16H 40/67, G06Q 20/14, G06Q 10/08, G06Q 30/04, G16H 40/63, G16H 40/20

(54) **INVENTORY MANAGEMENT AND LOCATION TRACKING OF MEDICAL DEVICES**
BESTANDSVERWALTUNG UND STANDORTVERFOLGUNG VON MEDIZINISCHEN VORRICHTUNGEN
GESTION ET ADMINISTRATION DE DISPOSITIFS MÉDICAUX ET SUIVI DE LEUR LOCALISATION

(30) Priority: 31.07.2014 US 201462031394 P
(43) Date of publication of application: 07.06.2017
(73) Proprietor: Smith & Nephew, Inc, Memphis, TN 38116 (US)
(72) Inventor: FOWLER, Alex, Hull HU3 2BN (GB); YEAMAN, Annaliese, Hull HU3 2BN (GB)
(74) Representative: Smith & Nephew
(86) International application number: PCT/US2015/043004
(87) International publication number: WO 2016/019191

(56) References cited:
- WO-A1-2012/057881
- WO-A2-2009/151645
- WO-A2-2014/151930
- WO-A2-2015/073809
- US-A1- 2010 022 990

## Description

### CROSS-REFERENCE TO RELATED APPLICATIONS

This application claims the benefit of U.S. Provisional Application No. 62/031,394, filed July 31, 2014.

### BACKGROUND

### Field

Embodiments of the present disclosure relate to methods and apparatuses for dressing and treating a wound with reduced pressure therapy or topical negative pressure (TNP) therapy. In particular, embodiments disclosed herein relate to negative pressure therapy devices with location communication and related methods. An example of a prior art apparatus and method is disclosed in US2010/0022990A1.

### Description of the Related Art

Many different types of wound dressings are known for aiding in the healing process of a human or animal. These different types of wound dressings include many different types of materials and layers, for example, gauze, pads, foam pads or multilayer wound dressings. Topical negative pressure (TNP) therapy, sometimes referred to as vacuum assisted closure, negative pressure wound therapy, or reduced pressure wound therapy, is widely recognized as a beneficial mechanism for improving the healing rate of a wound. Such therapy is applicable to a broad range of wounds such as incisional wounds, open wounds and abdominal wounds or the like.

TNP therapy assists in the closure and healing of wounds by reducing tissue oedema, encouraging blood flow, stimulating the formation of granulation tissue, removing excess exudates and may reduce bacterial load and, thus, infection to the wound. Furthermore, TNP therapy permits less outside disturbance of the wound and promotes more rapid healing.

### SUMMARY

The invention is defined by the appended claims.

### BRIEF DESCRIPTION OF THE DRAWINGS

Embodiments of the present disclosure will now be described hereinafter, by way of example only, with reference to the accompanying drawings in which:
Figure 1 illustrates a reduced pressure wound therapy system according to some embodiments.
Figure 2A, 2B, and 2C illustrate electrical component schematics of pump assemblies according to some embodiments.
Figure 3 illustrates a system for location monitoring according to some embodiments.

### DETAILED DESCRIPTION OF SOME EMBODIMENTS

### Introduction

Embodiments disclosed herein relate to systems and methods of treating a wound with reduced pressure. As is used herein, reduced or negative pressure levels, such as -X mmHg, represent pressure levels relative to normal ambient atmospheric pressure, which can correspond to 760 mmHg (or 1 atm, 29.93 inHg, 101.325 kPa, 14.696 psi, etc.). Accordingly, a negative pressure value of -X mmHg reflects absolute pressure that is X mmHg below 760 mmHg or, in other words, an absolute pressure of (760-X) mmHg. In addition, negative pressure that is "less" or "smaller" than X mmHg corresponds to pressure that is closer to atmospheric pressure (for example, -40 mmHg is less than -60 mmHg). Negative pressure that is "more" or "greater" than -X mmHg corresponds to pressure that is further from atmospheric pressure (for example, -80 mmHg is more than -60 mmHg). In some embodiments, local ambient atmospheric pressure is used as a reference point, and such local atmospheric pressure may not necessarily be, for example, 760 mmHg.

Embodiments of the present disclosure are generally applicable to use in topical negative pressure (TNP) or reduced pressure therapy systems. Briefly, negative pressure wound therapy assists in the closure and healing of many forms of "hard to heal" wounds by reducing tissue oedema, encouraging blood flow and granular tissue formation, and/or removing excess exudate and can reduce bacterial load (and thus infection risk). In addition, the therapy allows for less disturbance of a wound leading to more rapid healing. TNP therapy systems can also assist in the healing of surgically closed wounds by removing fluid. In some embodiments, TNP therapy helps to stabilize the tissue in the apposed position of closure. A further beneficial use of TNP therapy can be found in grafts and flaps where removal of excess fluid is important and close proximity of the graft to tissue is required in order to ensure tissue viability.

The provider or manufacturer of TNP or reduced pressure therapy systems, such as pump assemblies, can desire to bill for the possession or usage of pump assemblies. The process of accounting for possession or use of the pump assemblies, however, can be difficult for the provider to manage since the provider may not have control over the administration and distribution of the pump assemblies. The provider may rely on other parties, such as hospital staff, to accurately track the possession or use of the pump assemblies. The other parties, unfortunately, may not at times accurately track the possession or use of the pump assemblies, so the provider may rely on erroneous or incomplete information from the other parties when accounting for and subsequently billing for the usage of pump assemblies. This situation can risk over or under billing for use of the pump assemblies. Accordingly, disclosed systems and methods can assist the provider of pump assemblies in accurately monitoring and tracking the pump assemblies to account for possession or use of the pump assemblies. Disclosed systems and methods are also more generally applicable to asset tracking of any type of inventory.

### Negative Pressure System

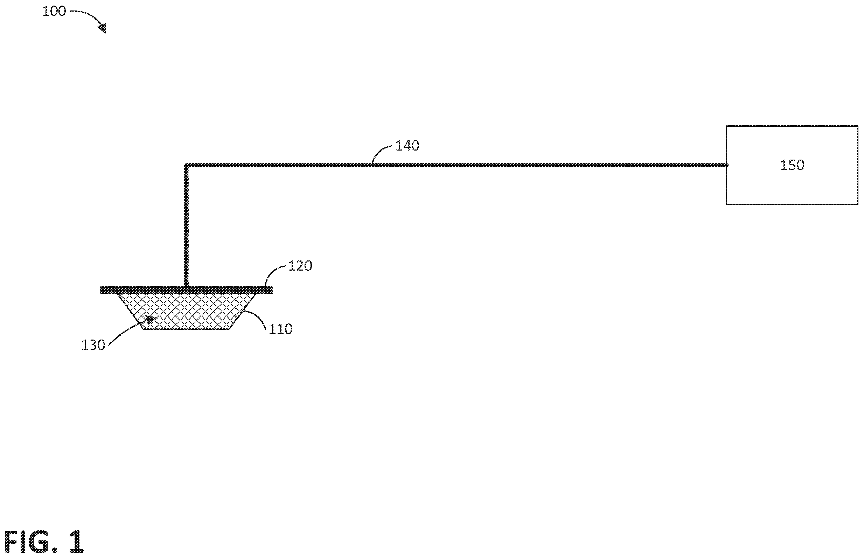

Figure 1 illustrates an embodiment of a negative or reduced pressure wound treatment (or TNP) system 100 comprising a wound filler 130 placed inside a wound cavity 110, the wound cavity sealed by a wound cover 120. The wound filler 130 in combination with the wound cover 120 can be referred to as wound dressing. A single or multi lumen tube or conduit 140 is connected the wound cover 120 with a pump assembly 150 configured to supply reduced pressure. The wound cover 120 can be in fluidic communication with the wound cavity 110. In any of the system embodiments disclosed herein, as in the embodiment illustrated in Figure 1, the pump assembly 150 can be a canisterless pump assembly (meaning that exudate is collected in the wound dressing or is transferred via tube 140 for collection to another location). However, any of the pump assembly embodiments disclosed herein can be configured to include or support a canister. Additionally, in any of the system embodiments disclosed herein, any of the pump assembly embodiments can be mounted to or supported by the dressing, or adjacent to the dressing. The wound filler 130 can be any suitable type, such as hydrophilic or hydrophobic foam, gauze, inflatable bag, and so on. The wound filler 130 can be conformable to the wound cavity 110 such that it substantially fills the cavity. The wound cover 120 can provide a substantially fluid impermeable seal over the wound cavity 110. The wound cover 120 can have a top side and a bottom side, and the bottom side adhesively (or in any other suitable manner) seals with wound cavity 110. The conduit 140 or lumen or any other conduit or lumen disclosed herein can be formed from polyurethane, PVC, nylon, polyethylene, silicone, or any other suitable material.

Some embodiments of the wound cover 120 can have a port (not shown) configured to receive an end of the conduit 140. In other embodiments, the conduit 140 can otherwise pass through and/or under the wound cover 120 to supply reduced pressure to the wound cavity 110 so as to maintain a desired level of reduced pressure in the wound cavity. The conduit 140 can be any suitable article configured to provide at least a substantially sealed fluid flow pathway between the pump assembly 150 and the wound cover 120, so as to supply the reduced pressure provided by the pump assembly 150 to wound cavity 110.

The wound cover 120 and the wound filler 130 can be provided as a single article or an integrated single unit. In some embodiments, no wound filler is provided and the wound cover by itself may be considered the wound dressing. The wound dressing may then be connected, via the conduit 140, to a source of negative pressure, such as the pump assembly 150. The pump assembly 150 can be miniaturized and portable, although larger conventional pumps such can also be used.

The wound cover 120 can be located over a wound site to be treated. The wound cover 120 can form a substantially sealed cavity or enclosure over the wound site. In some embodiments, the wound cover 120 can be configured to have a film having a high water vapour permeability to enable the evaporation of surplus fluid, and can have a superabsorbing material contained therein to safely absorb wound exudate. It will be appreciated that throughout this specification reference is made to a wound. In this sense it is to be understood that the term wound is to be broadly construed and encompasses open and closed wounds in which skin is torn, cut or punctured or where trauma causes a contusion, or any other surficial or other conditions or imperfections on the skin of a patient or otherwise that benefit from reduced pressure treatment. A wound is thus broadly defined as any damaged region of tissue where fluid may or may not be produced. Examples of such wounds include, but are not limited to, acute wounds, chronic wounds, surgical incisions and other incisions, subacute and dehisced wounds, traumatic wounds, flaps and skin grafts, lacerations, abrasions, contusions, burns, diabetic ulcers, pressure ulcers, stoma, surgical wounds, trauma and venous ulcers or the like.

Some embodiments of the system 100 are designed to operate without the use of an exudate canister. Some embodiments can be configured to support an exudate canister. In some embodiments, configuring the pump assembly 150 and tubing 140 so that the tubing 140 can be quickly and easily removed from the pump assembly 150 can facilitate or improve the process of dressing or pump changes, if necessary. Any of the pump embodiments disclosed herein can be configured to have any suitable connection between the tubing 140 and the pump assembly 150.

In some embodiments, the pump assembly 150 can be configured to deliver negative pressure of approximately -80 mmHg, or between about -20 mmHg and -200 mmHg. Note that these pressures are relative to normal ambient atmospheric pressure thus, -200 mmHg would be about 560 mmHg in practical terms. The pressure range can be between about -40 mmHg and -150 mmHg. Alternatively a pressure range of up to -75 mmHg, up to -80 mmHg or over -80 mmHg can be used. Also a pressure range of below -75 mmHg can be used. Alternatively a pressure range of over approximately -100 mmHg, or even 150 mmHg, can be supplied by the pump assembly 150.

In some embodiments, the pump assembly 150 is configured to provide continuous or intermittent negative pressure therapy. During intermittent therapy, negative pressure at low setpoint can be delivered for a first time duration, and upon expiration of the first time duration, negative pressure at high setpoint can be delivered for a second time duration. Upon expiration of the second time duration, negative pressure at low setpoint can be delivered. The first and second time durations can be same or different values.

In operation, the wound filler 130 is inserted into the wound cavity 110 and wound cover 120 is placed so as to seal the wound cavity 110. The pump assembly 150 provides a source of a negative pressure to the wound cover 120, which is transmitted to the wound cavity 110 via the wound filler 130. Fluid (for example, wound exudate) is drawn through the conduit 140, and can be stored in a canister. In some embodiments, fluid is absorbed by the wound filler 130 or one or more absorbent layers (not shown).

Wound dressings that may be utilized with the pump assembly and other embodiments of the present application include Renasys-F, Renasys-G, Renasys AB, and Pico Dressings available from Smith & Nephew. Further description of such wound dressings and other components of a negative pressure wound therapy system that may be used with the pump assembly and other embodiments of the present application are found in U.S. Patent Publication Nos. 2011/0213287, 2011/0282309, 2012/0116334, 2012/0136325, and 2013/0110058. In other embodiments, other suitable wound dressings can be utilized.

### Electronics and Software

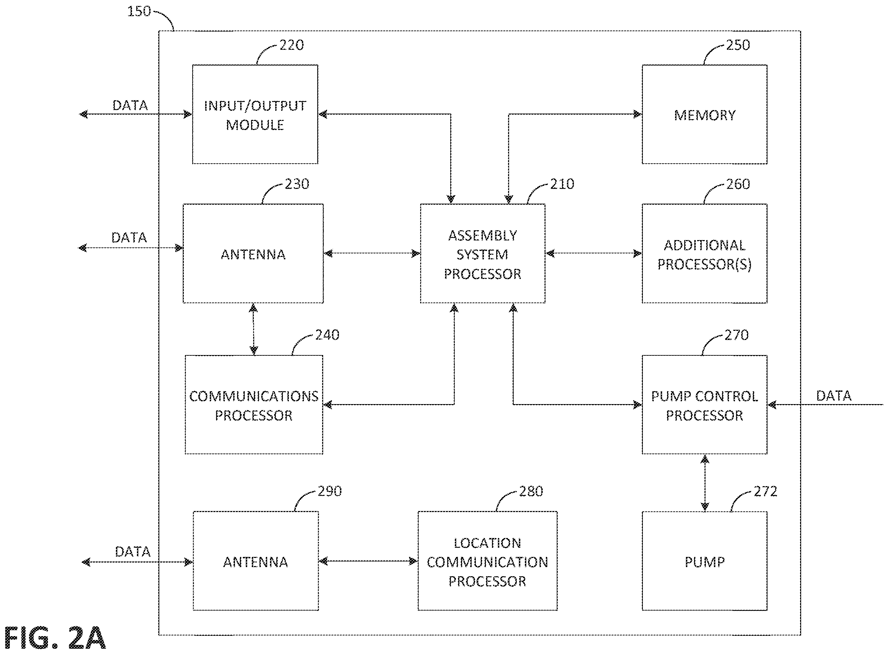

Figure 2A illustrates an electrical component schematic of the pump assembly 150 of Figure 1, according to some embodiments. Electrical components of the pump assembly 150 can be included as part of a pump assembly housing (for example, within or attached to the pump assembly housing), operate to accept user input, provide output to the user, operate the pump assembly 150 and the TNP system, provide network connectivity, and so on. Electrical components of the pump assembly 150 can be mounted on one or more printed circuit boards (PCBs).

The pump assembly 150 can include an assembly system processor 210 configured to control operation of one or more components of the pump assembly 150. Input to and output from the pump assembly 150 can controlled by an input/output (I/O) module 220. For example, the I/O module 220 can receive and transmit data from one or more ports, such as serial, parallel, hybrid ports, and the like. The processor 210, along with other controllers or processors of the pump assembly 150, may store data in a memory 250, which can be internal and/or external to the processor 210. Any suitable type of memory can be used, including volatile and/or non-volatile memory, such as one or more of random-access memory (RAM), read-only memory (ROM), magnetic memory, solid-state memory, magnetoresistive RAM (MRAM), and the like.

In some embodiments, the processor 210 can be a general purpose controller, such as a low-power processor. In other embodiments, the processor 210 can be an application specific processor. The processor 210 can be configured as a "central" processor in the electronic architecture of the pump assembly 150, and the processor 210 can coordinate the activity of other processors, such as a pump control processor 270, communications processor 230, and one or more additional processors 260 (for example, processor for controlling a display, buttons, and the like). The processor 210 can run a suitable operating system, such as a Linux™, Windows™ CE, VxWorks™, and the like.

The pump control processor 270 can be configured to control the operation of a negative pressure pump 272. The negative pressure pump 272 can be a suitable pump, such as a diaphragm pump, peristaltic pump, rotary pump, rotary vane pump, scroll pump, screw pump, liquid ring pump, diaphragm pump operated by a piezoelectric transducer, voice coil pump, and the like. The pump control processor 270 can measure pressure in a fluid flow path, using data received from one or more pressure sensors, calculate the rate of fluid flow, and control the pump. The pump control processor 270 can control a pump motor so that a desired level of negative pressure is achieved in the wound cavity 110. The desired level of negative pressure can be pressure set or selected by the user. In various embodiments, the pump control processor 270 controls the pump (for example, pump motor) using pulse-width modulation (PWM). A control signal for driving the pump can be a 0-100% duty cycle PWM signal. The pump control processor 270 can perform flow rate calculations and detect various conditions in a flow path. The pump control processor 270 can communicate information to the processor 210. The pump control processor 370 can include internal memory and/or can utilize memory 250. The pump control processor 270 can be a low-power processor.

A communications processor 240 can be configured to provide wired and/or wireless connectivity. The communications processor 240 can utilize one or more antennas 230 for sending and receiving data. The communications processor 240 can provide one or more of the following types of connections: Global Positioning System (GPS) technology, cellular connectivity (for example, 2G, 3G, LTE, 4G), WiFi™ connectivity, Internet connectivity, Bluetooth™ connectivity, and the like. Connectivity can be used for various activities, such as pump assembly location tracking, asset tracking, compliance monitoring, remote selection, uploading of logs, alarms, and other operational data, and adjustment of therapy settings, upgrading of software and/or firmware, and the like. The communications processor 340 can provide dual GPS/cellular functionality. Cellular functionality can, for example, be 3G functionality. In such cases, if the GPS module is not able to establish satellite connection due to various factors including atmospheric conditions, building or terrain interference, satellite geometry, and so on, the device location can be determined using the 3G network connection, such as by using cell identification, triangulation, forward link timing, and the like. The pump assembly 150 can include a SIM card, and SIM-based positional information can be obtained.

The communications processor 240 can communicate information to the processor 240. The communications processor 240 can include internal memory and/or can utilize memory 250. The communications processor 240 can be a low-power processor.

In some embodiments, the pump assembly 150 can track and store various data, such as one or more of positioning data, therapy parameters, logs, device data, and so on. The pump assembly 150 can track and log therapy and other operational data. Data can be stored, for example, in the memory 250.

In some embodiments, using the connectivity provided by the communications processor 240, the pump assembly 150 can upload any of the data stored, maintained, and/or tracked by the pump assembly. For example, the following information can be uploaded to a remote computer or server: activity log(s), which includes therapy delivery information, such as therapy duration, alarm log(s), which includes alarm type and time of occurrence; error log, which includes internal error information, transmission errors, and the like; therapy duration information, which can be computed hourly, daily, and the like; total therapy time, which includes therapy duration from first applying a particular therapy program or programs; lifetime therapy information; device information, such as the serial number, software version, battery level, and the like; device location information; patient information; and so on. The pump assembly 150 can also download various operational data, such as therapy selection and parameters, firmware and software patches and upgrades, and the like. The pump assembly 150 can provide Internet browsing functionality using one or more browser programs, mail programs, application software, and the like.

The pump assembly 150 can further include a location communication processor 280 and an antenna 290. The location communication processor 280 can be a transmitter or transmitter-receiver and use the antenna 290 to communicate a location of the pump assembly 150, such as a location of the housing of the pump assembly 150, to other devices in the proximity (for example, within 10, 20, or 50 meters and the like) of the pump assembly 150. The location communication processor 280 can perform one-way or two-way communication with the other devices depending on the implementation and utilize one or more of the following types of connections: cellular connectivity (for example, 2G, 3G, LTE, 4G), WiFi™ connectivity, Internet connectivity, Bluetooth™ connectivity, and the like. The communications transmitted by the location communication processor 280 can include identifying information to uniquely identify the pump assembly 150 relative to one or more other pump assemblies also in the proximity of the pump assembly 150. For example, identifying information can include a serial number or a value derived from the serial number. The signal strength of the transmitted communications by the location communication processor 280 can be controlled (for example, maintained at a constant or substantially constant level) to enable another device to determine a distance to the pump assembly 150, such as a distance between the device and the pump assembly 150. The location communication processor 280 and the antenna 290 can, in some implementations, have a power supply (not shown), such as a battery electrically coupled to a photo voltaic cell, separate from a power supply used to power the other components of the pump assembly 150 and usable to power the location communication processor 280 and the antenna 290. In such implementations, the location communication processor 280 can remain operational when the pump assembly 150 is not powered.

In some embodiments, the location communication processor 280 can communicate with other devices in the proximity of the pump assembly 150 so that the location communication processor 280 can itself determine a distance from the pump assembly 150 to the other devices. The location communication processor 280, in such embodiments, can track and store the distance from the pump assembly 150 to the other devices or indications of change in the distance over time, and the location communication processor 280 can later provide this information to the other devices. For instance, the location communication processor 280 can determine a duration of time during which the pump assembly 150 has been removed from a coverage area of a device and subsequently report this time to the device upon being returned to the coverage area.

As shown in Figure 2A, the location communication processor 280 and the antenna 290 can be disposed within the housing of the pump assembly 150. The location communication processor 280 and the antenna 290 can be configured to together be inserted and removed from the inside of the housing of the pump assembly 150. For example, to facilitate easy installation and removal, the location communication processor 280 and the antenna 290 can be part of a common housing or single integrated unit that may be placed within the housing of the pump assembly 150. The common housing or single integrated unit can also include a power supply (not shown) separate from a power supply used to power the other components of the pump assembly 150 and usable to power the location communication processor 280 and the antenna 290. The power supply of the common housing or single integrated unit can enable the location communication processor 280 and the antenna 290 to function independently from the operations or power supply of the other components of the pump assembly 150. Existing pump assemblies can moreover be outfitted or retrofitted with the functionality of the location communication processor 280 and the antenna 290. For example, an existing pump assembly can be retrofitted with the functionality by individually placing such common housings or single integrated units within or on the surface of the existing pump assemblies. In some embodiments, the location communication processor 280 may operate without the antenna 290.

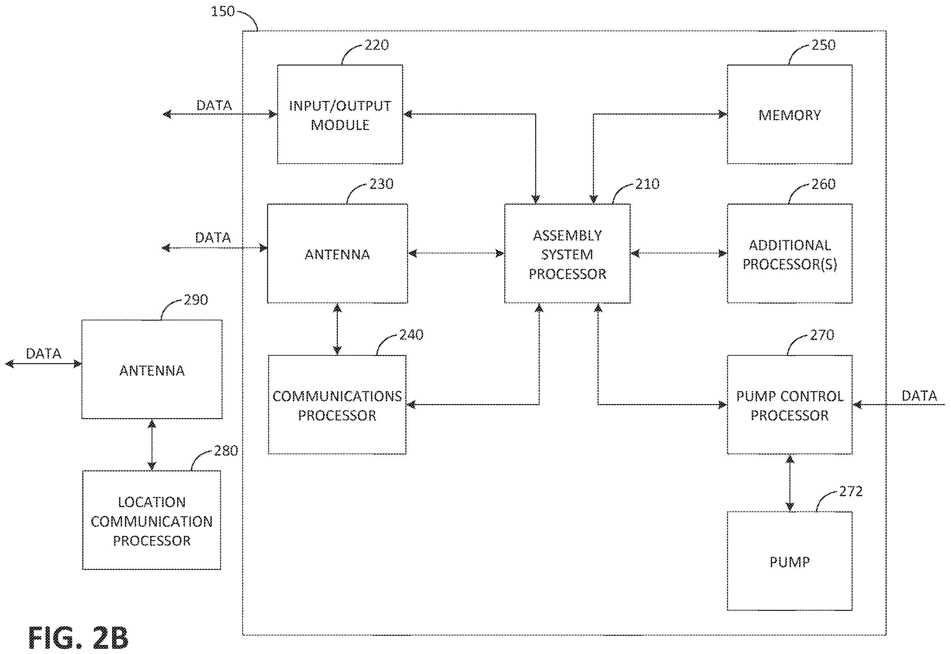

As shown in Figure 2B, the location communication processor 280 and the antenna 290 can be disposed outside the housing of the pump assembly 150. The location communication processor 280 and the antenna 290 can be configured to together be attached and removed from an external surface of the housing of the pump assembly 150. For example, to facilitate easy installation and removal, the location communication processor 280 and the antenna 290 can be part of a common housing or single integrated unit that may be attached using glue, tape, fastener, and the like to the external surface of the housing. The common housing or single integrated unit can also include a power supply (not shown) separate from a power supply used to power the other components of the pump assembly 150 and usable to power the location communication processor 280 and the antenna 290. The power supply of the common housing or single integrated unit can enable the location communication processor 280 and the antenna 290 to function independently from the operations or power supply of the other components of the pump assembly 150. Existing pump assemblies can accordingly be outfitted or retrofitted with the functionality of the location communication processor 280 and the antenna 290 by coupling or attaching such common housings or single integrated units to the existing pump assemblies.

When the location communication processor 280 and the antenna 290 are part of the common housing or single integrated unit (for instance, as described with respect to certain examples of Figures 2A and 2B), the common housing or single integrated unit can be electrically isolated from the other components of the pump assembly 150. The common housing or single integrated unit thus may not be in electrical communication with the other components of the pump assembly 150, and the location communication processor 280 and the antenna 290 may not electrically transmit or receive data (for example, operation instructions or information related to the provision of negative therapy) or power to or from the other components of the pump assembly 150, like the processor 210, pump control processor 270, and communications processor 240.

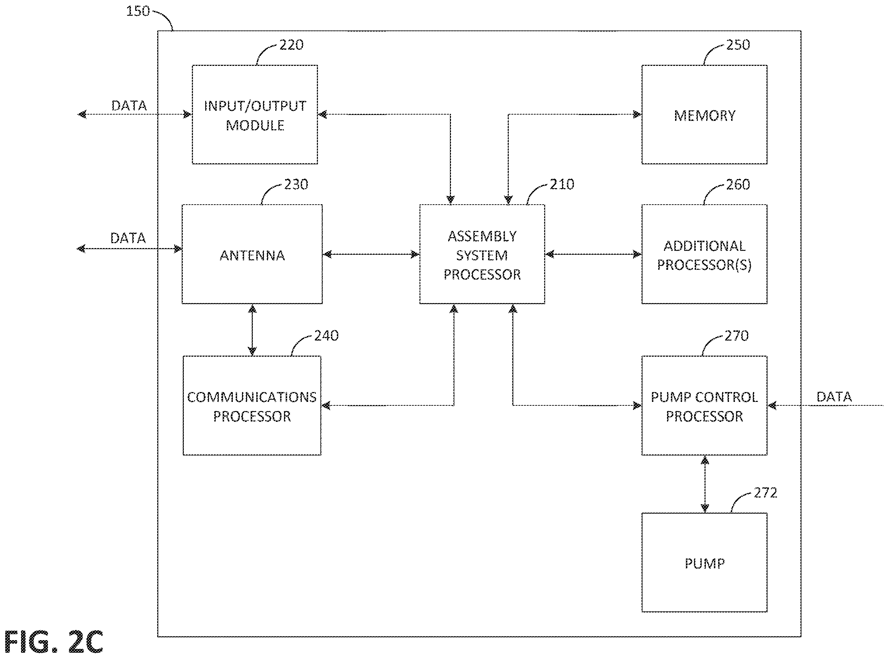

The pump assembly 150 shown in Figure 2C can be similar to the pump assembly 150 of Figure 2A, except that the functions of the location communication processor 280 and the antenna 290 can respectively be performed by the communications processor 240 and the antenna 230. As a result, the pump assembly 150 of Figure 2C may not have separate location communication circuitry for performing location communications as in the pump assembly 150 of Figures 2A or 2B.

### Example Location Monitoring System

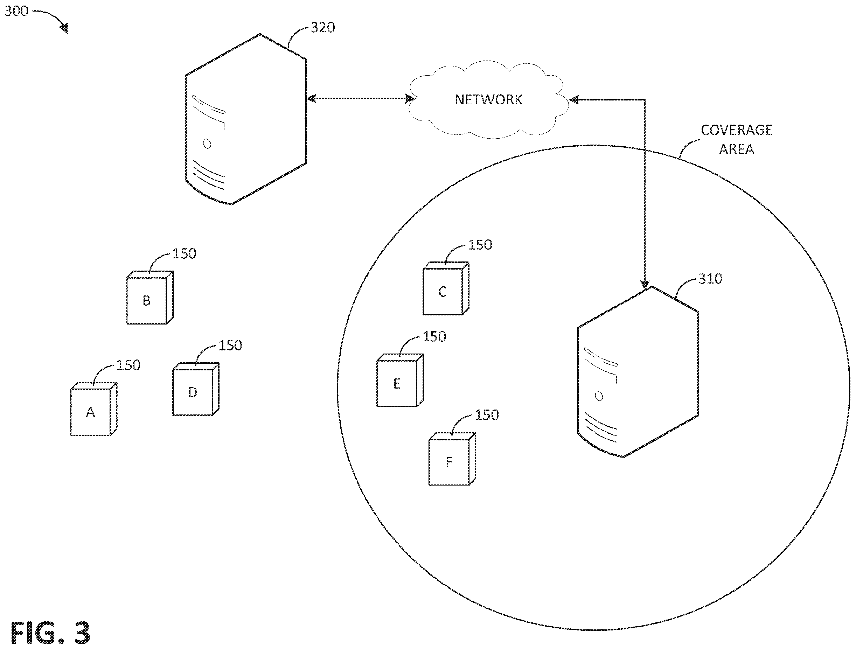

Figure 3 illustrates a system 300 for monitoring the locations of pump assemblies according to some embodiments. The system 300 includes multiple pump assemblies 150 and a location monitoring hub 310. The multiple pump assemblies 150 can each be, for example, one of the pump assemblies 150 described with respect to Figures 2A-2C. The location monitoring hub 310 can communicate with the multiple pump assemblies 150 to individually monitor the locations of the multiple pump assemblies 150. Based on the determined locations of the multiple pump assemblies 150, the location monitoring hub 310 can automatically determine whether the multiple pump assemblies 150 may be within a proximity or a coverage area of the location monitoring hub 310 and thereby control inventory management related to the multiple pump assemblies 150, such as in connection with billing for the use of the pump assemblies 150. The location monitoring hub 310 can utilize one or more of the following types of connections: cellular connectivity (for example, 2G, 3G, LTE, 4G, GPRS), WiFi™ connectivity, WLAN connectivity, Internet connectivity, Bluetooth™ connectivity, ZigBee connectivity, and the like.

Individual pump assemblies of the multiple pump assemblies 150 can repeatedly communicate with the location monitoring hub 310 to repeatedly indicate to the location monitoring hub 310 whether the multiple pump assemblies 150 may be present in the proximity of the location monitoring hub 310. The pump assembly A can, for instance, transmit a signal using a Bluetooth™ protocol communication to the location monitoring hub 310 on a periodic, random, or scheduled basis (for instance, every 1, 5, or 20 seconds) and the like indicating that the pump assembly A may be in the proximity of the location monitoring hub 310. In one implementation, the pump assembly A can transmit the signal with a frequency based at least on a minimum billing period of the pump assembly A, such that the pump assembly A transmits the signal at least once per minimum billing period. For example, if the minimum billing period for the pump assembly A is 60 minutes, the pump assembly A can transmit the signal with a 30 minute periodicity. The location monitoring hub 310 can, in turn, use the received signal from the pump assembly A to determine that the pump assembly A is present in the proximity of the location monitoring hub 310. The location monitoring hub 310 can also use the received signal to determine the change in location over time of the pump assembly A relative to the location monitoring hub 310.

The location monitoring hub 310 can determine the location of individual pump assemblies of the multiple pump assemblies 150 over time. In one example, the location monitoring hub 310 can determine the location of an individual pump assembly, such as the pump assembly A, based at least on whether the location monitoring hub 310 received a communication from the individual pump assembly recently (for example, within a threshold period of time). When a communication has not been received recently, the location monitoring hub 310 can conclude or establish that the individual pump assembly is not within the proximity of the location monitoring hub 310. In such cases, the location monitoring hub 310 may receive additional communications or information from the individual pump assembly indicating whether further communication may not be received for other reasons, such as if a low battery condition at the individual pump assembly may cause the individual pump assembly to shut down and cease communications. The additional communications or information can be used by the location monitoring hub 310 to also indicate to send out an engineer to repair or replace the individual pump assembly. In another example, the location monitoring hub 310 can determine the location of an individual pump assembly, such as the pump assembly B, based at least on the signal strength of a received communication from the individual pump assembly at the location monitoring hub 310. As the signal strength of the received communication diminishes, the location monitoring hub 310 can determine that the individual pump assembly is farther from the location monitoring hub 310. In some embodiments, the location monitoring hub 310 can include two or more antennas usable to receive communications from the multiple pump assemblies 150, enabling the signal strength at the individual antennas to be used to more precisely determine (for example, triangulate) the locations of the multiple pump assemblies 150.

The location monitoring hub 310 can perform or facilitate inventory management functions for the multiple pump assemblies 150 based on the coverage area for the location monitoring hub 310. The coverage area can be a geographical area being monitored by the location monitoring hub 310, which can be used to make decisions about the status of the multiple pump assemblies 150. For example, the coverage area of the location monitoring hub 310 can correspond to the boundaries of a medical device storage facility, such as a storage closet in a hospital. When the location monitoring hub 310 determines that an individual pump assembly is located within the coverage area, it may be concluded that the individual pump assembly is stored in the inventory storage area and not currently out for use by a patient. On the other hand, when the location monitoring hub 310 determines that an individual pump assembly is outside the coverage area, it can be concluded that the individual pump assembly is currently out for use by a patient such that the provider of the individual pump assembly can begin billing for the use of the individual pump assembly.

The location monitoring hub 310 can further facilitate management of inventory levels across different coverage areas. For example, if most or all of the pump assemblies in a particular coverage area may have been removed for use, the location monitoring hub 310 can automatically indicate to send additional pump assemblies to the particular coverage area. Alternatively, if a number of pump assemblies in a certain coverage area may remain unused for an extended period of time, the location monitoring hub 310 can automatically indicate to redistribute some of the pump assemblies in the certain coverage area to another coverage area.

As illustrated by Figure 3, the coverage area can be an area around the location monitoring hub 310, such as an area defined by any location within a certain distance from the location monitoring hub 310. For example, the coverage area can be circular or spherical with the location monitoring hub 310 positioned at the center. In other embodiments, the coverage area can be a non-circular or asymmetrical area located around the location monitoring hub 310 or some area monitored by the location monitoring hub 310 but not located around or near the location monitoring hub 310. The coverage area can be in part defined by a two- or three-dimensional region, such as a floor space area, which has an area, for example, of around 100 m², 500 m², or 1000 m², and the like. This can provide for geo-fencing capabilities.

The size or position of the coverage area can be controlled or set by the location monitoring hub 310 in some implementations. For example, a manager of the location monitoring hub 310 can input a desired size of the coverage area, and the location monitoring hub 310 can provide a coverage area having the desired size. In addition, the boundaries of the coverage area can depend on the range over which the location monitoring hub 310 can successfully communicate with the multiple pump assemblies 150. For instance, the range over which the location monitoring hub 310 can communicate with an individual pump assembly may define the coverage area for the location monitoring hub 310. In such instances, the range can, for example, be (1) a range over which the location monitoring hub 310 can receive communications from the multiple pump assemblies 150 without errors or (2) a range over which the location monitoring hub 310 can receive communications having a signal strength that exceeds a signal strength threshold.

In one implementation, the location monitoring hub 310 can monitor the locations of the multiple pump assemblies 150 relative to the coverage area over time and thus be used to indicate whether to bill for the multiple pump assemblies 150. The location monitoring hub 310 can be placed in a hospital storage area (for example, a storage closet or room) used for storing available-for-use pump assemblies, such as the pump assemblies C, E, and F. The coverage area, in turn, can span the hospital storage area so that the location monitoring hub 310 determines whether the multiple pump assemblies 150 are within or outside the hospital storage area. When a pump assembly, such as the pump assembly A, B, or D, is removed from the hospital storage area, the location monitoring hub 310 can infer that the pump assembly is being used for delivery of therapy to a patient such that the location monitoring hub 310 can indicate to begin billing for the removed pump assembly. As is illustrated in Figure 3, this indication can be provided to a remote computer 320 over any suitable network, such as the Internet. The remote computer 320 can be a billing system. Once the removed pump assembly is returned to the hospital storage area, the location monitoring hub 310 can conclude that the pump assembly is no longer in use and can indicate to stop billing for provision of negative therapy. This indication can also be provided to the remote computer 320. As a result, the location monitoring hub 310 can provide accurate indications of when to begin and stop billing for an individual pump assembly according at least to a comparison of when the individual pump assembly may have been removed from and returned to the hospital storage area. The indications can be provided using any suitable communication interface, such as by using iCloud technology. This can facilitate accurate tracking of usage and allow for accurate billing for delivery of negative pressure wound therapy, which in turn can facilitate accurate reimbursements from insurers.

In some embodiments, the system 300 can further be used to provide one or more checks to determine whether to bill for an individual pump assembly. For example, if an individual pump assembly is removed from and returned to the coverage area within a relatively short time (for example, within a time of less than 10 minutes), the removal and return timings for the individual pump assembly may be used to decide not to provide an indication to bill for the removal of the individual pump assembly. In another example, the location monitoring hub 310 can store an indication of whether a determined location for an individual pump assembly may be erroneous (for instance, a communicated message from the individual pump assembly may specify a location for the individual pump assembly different from the location of the individual pump assembly determined by the location monitoring hub 310), and thus may indicate not to bill for the individual pump assembly. In yet another example, an individual pump assembly can itself track timings or periods that the individual pump assembly may be outside the coverage area, and the timings or periods tracked by the individual pump assembly can be compared to the timings or time periods indicated by the location monitoring hub 310 for consistency. Moreover, other timings or periods tracked by an individual pump assembly (for instance, total therapy delivery time, device on time, activity timings in an activity log, and the like) can be compared to the timings or time period indicated by the location monitoring hub 310 to determine whether and when to bill for the individual pump assembly.

In some embodiments, the location monitoring hub 310 may be omitted as the individual pump assemblies can be configured to communicate directly with the remote computer 320 via the network. For instance, an individual pump assembly can provide its location directly to the remote computer 320 using the communications processor 240 or the location communication processor 280. The remote computer 320 can then determine whether the individual pump assembly may be within a coverage area based at least on the provided location.

### Other Variations

Although some embodiments of this disclosure are described using location communication for pump assemblies as examples, the location communication approaches described herein can be applied in other fields or used for location monitoring or asset tracking of other devices, such as other medical devices in a hospital (for instance, deep vein thrombosis (DVT) therapy devices, suction devices, continuous passive motion (CPM) devices, pacemaker devices, temperature management devices, and the like) or inventory or equipment in a warehouse. The approaches can be used, for instance, in fields in which the location of inventory may be tracked as being either within a zone or outside a zone. The approaches can enable more accurate understandings of the usage of the inventory and thus the more accurate accounting of costs associated with the inventory.

In one example, the location communication approaches described herein can be used to track devices such as compressors, drills, grinders, diagnostic systems, circuit testers, first aid kits, scanners, storage boxes, vacuums, projectors, and the like in one or more manufacturing warehouse storage rooms or areas. As the devices are removed from and returned to the storage rooms or areas, one or more location monitoring hubs can track the removal and returning of the devices and thus enable usage of the devices to be accounted for and inventory levels of the devices to be determined and managed. In another example, office equipment such as computers, phones, printers, and the like can be rented by a company under terms such that the company keeps a set of office equipment at the company office but pays rent for individual equipment of the set on a per usage basis. Accordingly, using the approaches provided herein, one or more office rooms or storage areas can be setup with one or more location monitoring hubs to monitor the removal and returning of individual equipment of the set to automatically control the billing for the individual equipment and manage the equipment inventory levels in the one or more office rooms or storage areas. In yet another example, a taxi company may rent a vehicle to a driver under terms such that the driver pays for use of the vehicle according to the time that the driver uses the vehicle. When the driver drives the vehicle from a taxi parking lot, a location monitoring hub disposed at the taxi parking lot and in communication with the vehicle can track the removal of the vehicle and indicate to begin billing for use of the vehicle. The location monitoring hub can additionally detect the return of the vehicle to the taxi parking lot and indicate to stop billing for use of the vehicle.

As used herein, an indication or to indicate can, in addition to having its ordinary meaning, respectively refer to a message or sending of a message via a communication channel (for instance, wired, wireless, electromagnetic, or magnetic mediums and the like) to point out information. The message can include data sufficient for a receiver of the message to determine the information pointed out in the message. In some implementations, the message or information pointed out in the message can cause the receiver or a device associated with the receiver to perform an action in accordance with the information pointed out in the message.

Any value of a threshold, limit, duration, etc. provided herein is not intended to be absolute and, thereby, can be approximate. In addition, any threshold, limit, duration, etc. provided herein can be fixed or varied either automatically or by a user. Furthermore, as is used herein relative terminology such as exceeds, greater than, less than, etc. in relation to a reference value is intended to also encompass being equal to the reference value. For example, exceeding a reference value that is positive can encompass being equal to or greater than the reference value. In addition, as is used herein relative terminology such as exceeds, greater than, less than, etc. in relation to a reference value is intended to also encompass an inverse of the disclosed relationship, such as below, less than, greater than, etc. in relations to the reference value.

Features, materials, characteristics, or groups described in conjunction with a particular aspect, embodiment, or example are to be understood to be applicable to any other aspect, embodiment or example described herein unless incompatible therewith. All of the features disclosed in this specification (including any accompanying example embodiments, abstract and drawings), and/or all of the steps of any method or process so disclosed, may be combined in any combination, except combinations where at least some of such features and/or steps are mutually exclusive. The protection is not restricted to the details of any foregoing embodiments. The protection extends to any novel one, or any novel combination, of the features disclosed in this specification (including any accompanying example embodiments, abstract and drawings), or to any novel one, or any novel combination, of the steps of any method or process so disclosed.

While certain embodiments have been described, these embodiments have been presented by way of example only, and are not intended to limit the scope of protection. Indeed, the novel methods and systems described herein may be embodied in a variety of other forms. Furthermore, various omissions, substitutions and changes in the form of the methods and systems described herein may be made. Those skilled in the art will appreciate that in some embodiments, the actual steps taken in the processes illustrated and/or disclosed may differ from those shown in the figures. Depending on the embodiment, certain of the steps described above may be removed, others may be added. For example, the actual steps and/or order of steps taken in the disclosed processes may differ from those shown in the figure. Depending on the embodiment, certain of the steps described above may be removed, others may be added. For instance, the various components illustrated in the figures may be implemented as software and/or firmware on a processor, controller, ASIC, FPGA, and/or dedicated hardware. Hardware components, such as processors, ASICs, FPGAs, and the like, can include logic circuitry. Furthermore, the features and attributes of the specific embodiments disclosed above may be combined in different ways to form additional embodiments, all of which fall within the scope of the present disclosure.

User interface screens illustrated and described herein can include additional and/or alternative components. These components can include menus, lists, buttons, text boxes, labels, radio buttons, scroll bars, sliders, checkboxes, combo boxes, status bars, dialog boxes, windows, and the like. User interface screens can include additional and/or alternative information. Components can be arranged, grouped, displayed in any suitable order.

## Claims

1. A negative pressure therapy apparatus comprising:
a housing (150) comprising a source of negative pressure configured to be in fluidic communication with a wound dressing (120), the source of negative pressure configured to apply negative pressure to the wound dressing;
a controller (210) disposed within the housing and configured to operate the source of negative pressure;
a transmitter and an antenna;
wherein the transmitter is configured to wirelessly communicate with a remote computing device when the apparatus is within a coverage area so as to enable the remote computing device to determine whether the apparatus is within the coverage area, the transmitter and the antenna being configured to function independently from operations or a power supply of the controller, wherein the transmitter and the antenna are configured to be powered by a power supply separate from the power supply of the controller; and
wherein the transmitter is configured to repeatedly wirelessly communicate with the remote computing device to cause the remote computing device to determine a first time when the apparatus is removed from the coverage area and a second time when the apparatus is returned to the coverage area, thereby causing the remote computing device to determine a duration of time that the apparatus is outside the coverage area based at least on a comparison of the first time and the second time.

2. The apparatus of claim 1, wherein the transmitter is configured to wirelessly communicate a signal using a substantially constant signal strength to enable the remote computing device to determine a location of the apparatus relative to the coverage area based at least on a signal strength of a signal received by the remote computing device from the transmitter.

3. The apparatus of claim 1, wherein the transmitter is configured to wirelessly transmit a signal that does not enable the remote computing device to detect a presence of the apparatus in the coverage area when the apparatus is positioned outside the coverage area.

4. The apparatus of claim 1, wherein the transmitter is configured to operate when the controller is not powered.

5. The apparatus of claim 1, wherein the transmitter is configured to wirelessly communicate a message to the remote computing device that indicates a low battery condition for a battery used to supply power to the transmitter.

6. The apparatus of claim 1, wherein the coverage area comprises an area of less than 1000 m² around a location of the remote computing device.

7. The apparatus of claim 1, wherein boundaries of the coverage area depend on a range over which the transmitter is capable of successfully communicating with the remote computing device.

8. The apparatus of claim 1, wherein the transmitter is configured to wirelessly communicate with the remote computing device using a Bluetooth protocol.

9. The apparatus of any of claims 1-8, wherein the transmitter is disposed within the housing.

10. The apparatus of claim 9, wherein the transmitter is configured to be removably connected to the housing.

11. The apparatus of claim 9, wherein the transmitter is configured to be retrofitted into the apparatus.

12. The apparatus of any of claims 1-8, wherein the transmitter is disposed on an external surface of the housing.

13. The apparatus of claim 12, wherein the transmitter is configured to be removably connected to the external surface.

14. A method of communicating the location of a negative pressure wound therapy apparatus, the method comprising:
providing a negative pressure wound therapy apparatus comprising a negative pressure source, a transmitter and an antenna wherein the apparatus further comprises a controller (210) disposed within a housing (150) of the negative pressure wound therapy apparatus and wherein the negative pressure source is operable to apply, via a flow path, negative pressure to a wound dressing (120); and
repeatedly wirelessly communicating with a remote computing device using the transmitter of the negative pressure wound therapy apparatus when the negative pressure wound therapy apparatus is within a coverage area so as to enable the remote computing device to determine whether the negative pressure wound therapy apparatus is within the coverage area, the transmitter and the antenna being configured to function independently from operations or a power supply of the controller, and wherein the transmitter and the antenna are configured to be powered by a power supply separate from the power supply of the controller.

15. The method of claim 14 wherein the negative pressure wound therapy apparatus is as defined in claim 1.

## Patentansprüche

1. Ein Unterdrucktherapiegerät, das Folgendes beinhaltet:
ein Gehäuse (150), das eine Unterdruckquelle beinhaltet, die konfiguriert ist, um in Fluidkommunikation mit einem Wundverband (120) zu stehen, wobei die Unterdruckquelle konfiguriert ist, um Unterdruck an den Wundverband anzulegen;
eine Steuerungseinheit (210), die innerhalb des Gehäuses angeordnet ist und konfiguriert ist, um die Unterdruckquelle zu betreiben;
einen Sender und eine Antenne;
wobei der Sender konfiguriert ist, um drahtlos mit einer externen Rechenvorrichtung zu kommunizieren, wenn sich das Gerät innerhalb eines Abdeckungsbereichs befindet, sodass der externen Rechenvorrichtung ermöglicht wird zu bestimmen, ob sich das Gerät innerhalb des Abdeckungsbereichs befindet, wobei der Sender und die Antenne konfiguriert sind, um unabhängig vom Betrieb oder einer Stromversorgung der Steuerungseinheit zu arbeiten, wobei der Sender und die Antenne konfiguriert sind, um durch eine Stromversorgung mit Strom versorgt zu werden, die von der Stromversorgung der Steuerungseinheit getrennt ist; und
wobei der Sender konfiguriert ist, um wiederholt drahtlos mit der externen Rechenvorrichtung zu kommunizieren, um zu bewirken, dass die externe Rechenvorrichtung einen ersten Zeitpunkt, an dem das Gerät aus dem Abdeckungsbereich entfernt wird, und einen zweiten Zeitpunkt, an dem das Gerät in den Abdeckungsbereich zurückgebracht wird, bestimmt, wodurch bewirkt wird, dass die externen Rechenvorrichtung eine Zeitdauer, während der sich das Gerät außerhalb des Abdeckungsbereichs befindet, basierend mindestens auf einem Vergleich des ersten Zeitpunkts und des zweiten Zeitpunkts bestimmt.

2. Gerät gemäß Anspruch 1, wobei der Sender konfiguriert ist, um drahtlos ein Signal unter Verwendung einer im Wesentlichen konstanten Signalstärke zu kommunizieren, um der externen Rechenvorrichtung zu ermöglichen, einen Standort des Geräts relativ zu dem Abdeckungsbereich basierend mindestens auf einer Signalstärke eines Signals von dem Sender, das durch die externe Rechenvorrichtung empfangen worden ist, zu bestimmen.

3. Gerät gemäß Anspruch 1, wobei der Sender konfiguriert ist, um drahtlos ein Signal zu übermitteln, das der externen Rechenvorrichtung nicht ermöglicht, eine Anwesenheit des Geräts in dem Abdeckungsbereich zu erfassen, wenn das Gerät außerhalb des Abdeckungsbereichs positioniert ist.

4. Gerät gemäß Anspruch 1, wobei der Sender konfiguriert ist, um betrieben zu werden, wenn die Steuerungseinheit nicht mit Strom versorgt wird.

5. Gerät gemäß Anspruch 1, wobei der Sender konfiguriert ist, um drahtlos an die externe Rechenvorrichtung eine Nachricht zu kommunizieren, die einen niedrigen Batterieladezustand für eine Batterie anzeigt, die verwendet wird, um den Sender mit Strom zu versorgen.

6. Gerät gemäß Anspruch 1, wobei der Abdeckungsbereich einen Bereich von weniger als 1000 m² um einen Standort der externen Rechenvorrichtung herum beinhaltet.

7. Gerät gemäß Anspruch 1, wobei Grenzen des Abdeckungsbereichs von einer Reichweite abhängen, über die der Sender in der Lage ist, erfolgreich mit der externen Rechenvorrichtung zu kommunizieren.

8. Gerät gemäß Anspruch 1, wobei der Sender konfiguriert ist, um drahtlos unter Verwendung eines Bluetooth-Protokolls mit der externen Rechenvorrichtung zu kommunizieren.

9. Gerät gemäß einem der Ansprüche 1-8, wobei der Sender innerhalb des Gehäuses angeordnet ist.

10. Gerät gemäß Anspruch 9, wobei der Sender konfiguriert ist, um entfernbar mit dem Gehäuse verbunden zu sein.

11. Gerät gemäß Anspruch 9, wobei der Sender konfiguriert ist, um in das Gerät nachgerüstet zu werden.

12. Gerät gemäß einem der Ansprüche 1-8, wobei der Sender auf einer äußeren Oberfläche des Gehäuses angeordnet ist.

13. Gerät gemäß Anspruch 12, wobei der Sender konfiguriert ist, um entfernbar mit der äußeren Oberfläche verbunden zu sein.

14. Ein Verfahren zum Kommunizieren des Standorts eines Unterdruckwundtherapiegeräts, wobei das Verfahren Folgendes beinhaltet:
Bereitstellen eines Unterdruckwundtherapiegeräts, das eine Unterdruckquelle, einen Sender und eine Antenne beinhaltet, wobei das Gerät ferner eine Steuerungseinheit (210) beinhaltet, die innerhalb eines Gehäuses (150) des Unterdruckwundtherapiegeräts angeordnet ist, und wobei die Unterdruckquelle betreibbar ist, um über einen Strömungsweg Unterdruck an einen Wundverband (120) anzulegen; und
wiederholtes drahtloses Kommunizieren mit einer externen Rechenvorrichtung unter Verwendung des Senders des Unterdruckwundtherapiegeräts, wenn das Unterdruckwundtherapiegerät sich innerhalb eines Abdeckungsbereichs befindet, sodass der externen Rechenvorrichtung ermöglicht wird zu bestimmen, ob sich das Unterdruckwundtherapiegerät innerhalb des Abdeckungsbereichs befindet, wobei der Sender und die Antenne konfiguriert sind, um unabhängig vom Betrieb oder einer Stromversorgung der Steuerungseinheit zu arbeiten, und wobei der Sender und die Antenne konfiguriert sind, um durch eine Stromversorgung mit Strom versorgt zu werden, die von der Stromversorgung der Steuerungseinheit getrennt ist.

15. Verfahren gemäß Anspruch 14, wobei das Unterdruckwundtherapiegerät wie in Anspruch 1 definiert ist.

## Revendications

1. Un appareil de thérapie par pression négative comprenant :
un boîtier (150) comprenant une source de pression négative configurée pour être en communication fluidique avec un pansement pour plaie (120), la source de pression négative étant configurée pour appliquer une pression négative sur le pansement pour plaie ;
un organe de commande (210) disposé à l'intérieur du boîtier et configuré pour faire marcher la source de pression négative ;
un émetteur et une antenne ;
où l'émetteur est configuré pour communiquer sans fil avec un dispositif informatique à distance lorsque l'appareil se trouve à l'intérieur d'une zone de couverture de manière à permettre au dispositif informatique à distance de déterminer si l'appareil est à l'intérieur de la zone de couverture, l'émetteur et l'antenne étant configurés pour fonctionner indépendamment d'opérations ou d'un bloc d'alimentation en courant de l'organe de commande, où l'émetteur et l'antenne sont configurés pour être alimentés par un bloc d'alimentation en courant distinct du bloc d'alimentation en courant de l'organe de commande ; et
où l'émetteur est configuré pour communiquer sans fil de manière répétée avec le dispositif informatique à distance afin d'amener le dispositif informatique à distance à déterminer un premier temps où l'appareil est retiré de la zone de couverture et un deuxième temps où l'appareil est ramené dans la zone de couverture, amenant de ce fait le dispositif informatique à distance à déterminer une durée de temps où l'appareil se trouve à l'extérieur de la zone de couverture sur la base au moins d'une comparaison du premier temps et du deuxième temps.

2. L'appareil de la revendication 1, où l'émetteur est configuré pour communiquer sans fil un signal en utilisant une intensité de signal substantiellement constante afin de permettre au dispositif informatique à distance de déterminer un emplacement de l'appareil relativement à la zone de couverture sur la base au moins d'une intensité de signal d'un signal reçu par le dispositif informatique à distance en provenance de l'émetteur.

3. L'appareil de la revendication 1, où l'émetteur est configuré pour émettre sans fil un signal qui ne permet pas au dispositif informatique à distance de détecter une présence de l'appareil dans la zone de couverture lorsque l'appareil est positionné à l'extérieur de la zone de couverture.

4. L'appareil de la revendication 1, où l'émetteur est configuré pour marcher lorsque l'organe de commande n'est pas alimenté.

5. L'appareil de la revendication 1, où l'émetteur est configuré pour communiquer sans fil un message au dispositif informatique à distance qui indique un état de batterie faible pour une batterie utilisée afin de délivrer une alimentation en courant à l'émetteur.

6. L'appareil de la revendication 1, où la zone de couverture comprend une zone de moins de 1 000 m² autour d'un emplacement du dispositif informatique à distance.

7. L'appareil de la revendication 1, où des limites de la zone de couverture dépendent d'une gamme sur laquelle l'émetteur est capable de communiquer avec succès avec le dispositif informatique à distance.

8. L'appareil de la revendication 1, où l'émetteur est configuré pour communiquer sans fil avec le dispositif informatique à distance en utilisant un protocole Bluetooth.

9. L'appareil de n'importe lesquelles des revendications 1 à 8, où l'émetteur est disposé à l'intérieur du boîtier.

10. L'appareil de la revendication 9, où l'émetteur est configuré pour être connecté au boîtier de manière à pouvoir être retiré.

11. L'appareil de la revendication 9, où l'émetteur est configuré pour être rétroinstallé dans l'appareil.

12. L'appareil de n'importe lesquelles des revendications 1 à 8, où l'émetteur est disposé sur une surface externe du boîtier.

13. L'appareil de la revendication 12, où l'émetteur est configuré pour être connecté à la surface externe de manière à pouvoir être retiré.

14. Un procédé de communication de l'emplacement d'un appareil de thérapie par pression négative sur plaie, le procédé comprenant le fait :
de fournir un appareil de thérapie par pression négative sur plaie comprenant une source de pression négative, un émetteur et une antenne où l'appareil comprend en sus un organe de commande (210) disposé à l'intérieur d'un boîtier (150) de l'appareil de thérapie par pression négative sur plaie et où la source de pression négative peut être amenée à marcher pour appliquer, par le biais d'une trajectoire de flux, une pression négative sur un pansement pour plaie (120) ; et
de communiquer sans fil de manière répétée avec un dispositif informatique à distance en utilisant l'émetteur de l'appareil de thérapie par pression négative sur plaie lorsque l'appareil de thérapie par pression négative sur plaie se trouve à l'intérieur d'une zone de couverture de manière à permettre au dispositif informatique à distance de déterminer si l'appareil de thérapie par pression négative sur plaie est à l'intérieur de la zone de couverture, l'émetteur et l'antenne étant configurés pour fonctionner indépendamment d'opérations ou d'un bloc d'alimentation en courant de l'organe de commande, et où l'émetteur et l'antenne sont configurés pour être alimentés par un bloc d'alimentation en courant distinct du bloc d'alimentation en courant de l'organe de commande.

15. Le procédé de la revendication 14 où l'appareil de thérapie par pression négative sur plaie est tel que défini dans la revendication 1.
